# EUROPEAN PATENT APPLICATION

(11) **EP 4 817 599 A1**
(43) Date of publication of application: **07.10.2026**
(21) Application number: 26166123.5
(22) Date of filing: 19.03.2026
(51) Int. Cl.: A61K 9/16, A61K 39/00, A61K 47/36, A61P 31/04, A61K 9/50, A61K 9/51

(54) **METHOD OF ENCAPSULATING MYCOBACTERIUM BOVIS BCG VACCINE BACILLI IN AN ALGINATE CARRIER TO COMBAT HELICOBACTER PYLORI INFECTION**

(30) Priority: 28.03.2025 PL 45160525
(71) Applicant: Uniwersytet Lodzki, 90-136 Lodz (PL); Centrum Badan Molekularnych i Makromolekularnych Polskiej Akademii Nauk z siedziba w Lodzi, 90363 Lodz (PL)
(72) Inventor: Gonciarz, Weronika, 91-728 ód (PL); Miko ajczyk-Chmiela, Magdalena, 91-496 ód (PL); Jaroniek, Patrycja, 92-547 ód (PL); Brzezi ski, Marek, 93-578 ód (PL)
(74) Representative: Kicinska-Fujawa, Alicja

(57) **Abstract**

The invention relates to a method of manufacturing microparticles containing *Mycobacterium bovis* BCG vaccine bacilli encapsulated in an alginate carrier, intended for combating *Helicobacter pylori* infection, characterised in that an aqueous solution of a non-ionic copolymeric surfactant (C₃H₆O.C₂H₄O)_{X} and/or a solution of chitosan in 1% acetic acid, and a suspension of live *M. bovis* BCG in 1 mL of saline solution with a BCG density of 3×10⁹ CFU/mL, and then subjected to a microencapsulation procedure by adding the above-mentioned solutions dropwise, using a 2 mL syringe, to a vessel containing a 0.5% calcium chloride solution, at room temperature on a magnetic stirrer for 1 hour. After one hour, the microcapsules are drained using a laboratory sieve and rinsed with sterile distilled water, using chitosans with a molecular weight in the range of 50.000-190.000 g/mol or 190.000-310.000 g/mol or 310.000-375.000 g/mol, with the concentration of the polymer solution used to prepare the alginate microcapsules being 2% v/v for alginate, 1% v/v for chitosan and 12 600 g/mol for the non-ionic copolymer surfactant (C₃H₆O.C₂H₄O)x.

## Description

The invention relates to a method for manufacturing microparticles containing *Mycobacterium bovis* BCG vaccine bacilli encapsulated in an alginate carrier, intended for the treatment of *Helicobacter pylori* infection.

Carriers for biologically active compounds should consist of biocompatible and biodegradable structural components and be non-toxic. These are the characteristics of alginate, which is used as a matrix for encapsulating active substances employed in the treatment of many lifestyle-related diseases, including infectious diseases. Furthermore, it is used as an ingredient in wound dressings, as it accelerates wound healing and promotes tissue regeneration [1]. It also has antibacterial and antiviral properties. Sodium alginate is used as an ingredient in medicines for heartburn and hyperacidity, as it neutralises the acidic pH of gastric juice.

Alginate is characterised by the presence of carboxyl (COOH) and hydroxyl (OH) groups, which allow for easy surface modification or functionalisation. Furthermore, alginates can easily be combined with various polymers to form composite films, membranes, microspheres, scaffolds and hydrogels, which serve as biocompatible materials for studying the interactions between cells and their microenvironment [2]. Furthermore, alginates do not adversely affect the viability of various types of cells [3].

Given the use of micro- or nanoparticles as carriers for the controlled delivery of drugs to the gastric or intestinal environment, it is necessary to modify them to account for the pH of the environment, which is acidic in the stomach and alkaline in the intestine.

A copolymer surfactant with the general formula (C₃H₆O·C₂H₄O)x, known by the trade name Pluronic^{®} F-127, hereinafter referred to as Pluronic, is known in the prior art.

The term *M. bovis BCG* as used in this description refers to the *Mycobacterium bovis BCG* vaccine bacilli. Live *M. bovis BCG* bacilli are also referred to as vaccine bacilli; they contain an attenuated but live strain of the bacterium *Mycobacterium bovis -* a closely related species that causes bovine tuberculosis. Therefore, the term *M. bovis BCG* may be used interchangeably with the term vaccine bacilli.

The use of alginate as a carrier requires maintaining a suitable pH level that does not cause it to degrade. Another technical challenge is maintaining the high viability of *M. bovis.*

In order for alginate-based carriers to meet these specific requirements, they should be sensitive to changes in the environment and release their cargo in the vicinity of the lesion or at the site where the cargo is intended to act on a specific type of cell, in order to achieve the desired biological effect.

To date, lipids have been the main substances used for the encapsulation of *Mycobacterium bovis* BCG [4]. In one of the methods, alginate was used to encapsulate *Mycobacterium bovis* BCG [5]; however, an emulsion method was employed, in which the aqueous phase consisted of a solution of alginate and *Mycobacterium bovis BCG,* and the oil phase consisted of olive oil, with the microparticles ultimately obtained through cross-linking with calcium chloride. The microparticles obtained using this method disintegrated after 40-60 minutes at physiological pH due to the lack of a surface coating with suitable polymers, such as chitosan or Pluronic F127.

The currently increasing antibiotic resistance of *H. pylori* is associated with the widespread overuse of antibiotics or their use not in accordance with physicians' recommendations, as well as the difficulty in achieving bactericidal antibiotic concentrations in the acidic pH of gastric juice. In 2018, the World Health Organization (WHO) stated that *H. pylori* resistance to antibiotics had reached an alarming level, and that clarithromycin-resistant isolates require the use of new therapeutic agents.

In view of the above, it is necessary to search for new substances to aid in the treatment of *H. pylori* infections, as well as modified methods of administration designed to ensure their release in the acidic environment of the stomach and to achieve maximum concentrations in the stomach, or to increase their resistance to the acidic environment of the stomach. It is also important to ensure that the active ingredients reach the small intestine and are released there at the highest possible concentration.

The inventors have developed an innovative method for producing a biopolymer carrier for *Mycobacterium bovis* BCG vaccine bacilli, in which an alginate carrier is modified.

Research has shown that modifying the surface of alginate with chitosan ensures the release of active substances in the acidic pH of gastric juice and provides suitable mucoadhesive properties. Alternatively, coating the alginate surface with a non-ionic copolymer surfactant, such as Pluronic^{®} F-127, provides protection against the adverse effects of acidic gastric juice. This strategy enables the production of alginate carriers in which both the rate of release of drugs or biologically active compounds can be controlled and their release triggered at the appropriate pH, whilst simultaneously protecting the components enclosed within the carrier from the effects of adverse environmental factors.

The invention relates to a method for producing microparticles containing *Mycobacterium bovis BCG* vaccine bacilli, characterised in that a non-ionic copolymeric surfactant (C₃H₆O.C₂H₄O)x and/or chitosans are introduced into an alginate solution, followed by a microencapsulation process, wherein the molecular weight of the chitosans lies within the range of 50.000-190.000 g/mol, 190.000-310.000 g/mol, or 310.000-375.000 g/mol, the concentration of the polymer solution used to prepare the alginate microcapsules is 2% by volume, whilst the concentration of the non-ionic copolymer surfactant (C₃H₆O.C₂H₄O)x is 12 600 g/mol.

Preferably, the method according to the invention uses live *M. bovis BCG* suspended in saline at a concentration of 3×10⁹ CFU/mL.

The microparticles obtained by the method of the invention enable the high viability of *M. bovis BCG* to be maintained. As demonstrated in the examples, the addition of a non-ionic copolymer surfactant (C₃H₆O.C₂H₄O)x ensures a survival rate of 96% for *M. bovis BCG,* whilst the addition of chitosan ensures a survival rate of approximately 95% for *M. bovis BCG.*

The microparticles obtained by the method of the invention are biocompatible *in vitro* and *in vivo.* Furthermore, chitosan-coated microparticles enhance the *in vitro* biocidal activity against *H. pylori.*

As demonstrated in the embodiments, the microparticles obtained by the method of the invention contain live *M. bovis BCG* bacilli, whose immunomodulatory properties are currently being intensively studied in the context of supporting the innate immune system in combating infectious agents, including both bacterial pathogens and viruses. *M. bovis* vaccine bacilli are used in many countries to confer immunity against tuberculosis or to support the treatment of bladder cancer [6]. The increase in the antimicrobial efficacy of macrophages following exposure to BCG bacilli or their components is likely the result of what is known as 'immune training' of these cells.

The presence of chitosan on the surface of microparticles enhances the biocidal effect against *H. pylori* bacteria by binding chitosan polysaccharides to lectins on the surface of *H. pylori.*

Once released from the microparticles modified with mucoadhesive chitosan, *M. bovis* BCG will, in the acidic pH of gastric juice (the site of *Helicobacter pylori* colonisation), bind to gastric mucins, blocking the attachment sites of *H. pylori,* and stimulate mucin production, thereby enabling the removal of *H. pylori* from their surface. This action by *M. bovis* BCG will result in a reduction in *H. pylori* colonisation. Furthermore, *M. bovis* BCG will activate tissue macrophages and other immunocompetent cells, including those present in the small intestine [7], in order to restore the phagocytic capacity impaired by *H. pylori,* which may contribute to the eradication of *H. pylori* infection.

The method described in the invention for encapsulating live *M. bovis* BCG in surface-modified alginate particles enables the gradual and controlled release of live *M. bovis* BCG at the appropriate pH and is significant for the use of the resulting biopolymers in the treatment of *H. pylori* infections. This is due to the colonisation of the human stomach by these bacteria. The resulting alginate carrier loaded with *M. bovis* BCG ensures that these bacteria are delivered as a biologically active component in their unaltered form, which is attributable to the carrier's solubility and/or its resistance to the acidic pH of gastric juice, which is approximately 3.0.

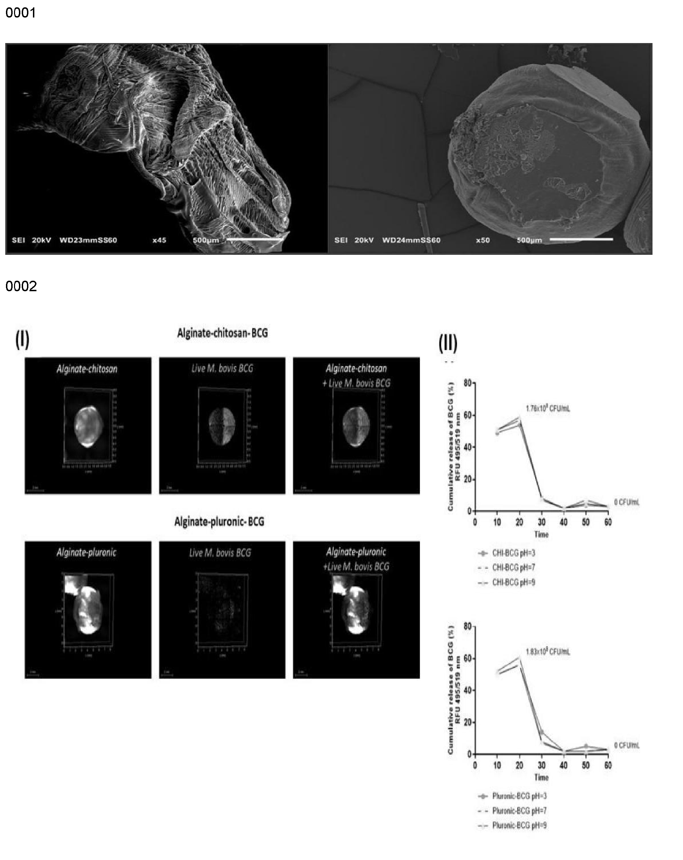

The invention is described in more detail in the examples and in the drawing, in which
[Fig. 1] shows example microscopic images of the microparticles obtained using a scanning electron microscope (SEM); on the left: ALG-Chitosan - chitosan-modified alginate particles and, on the right: ALG-Pluronic F127 - alginate particles modified with Pluronic^{®} F-127;
[Fig. 2] shows on the right: (I) example microscopic images showing the location of live *M. bovis* BCG bacilli within the prepared microparticles - hereinafter referred to as MCs, MCs (I); on the left (II) the kinetics of their release from the prepared MCs;

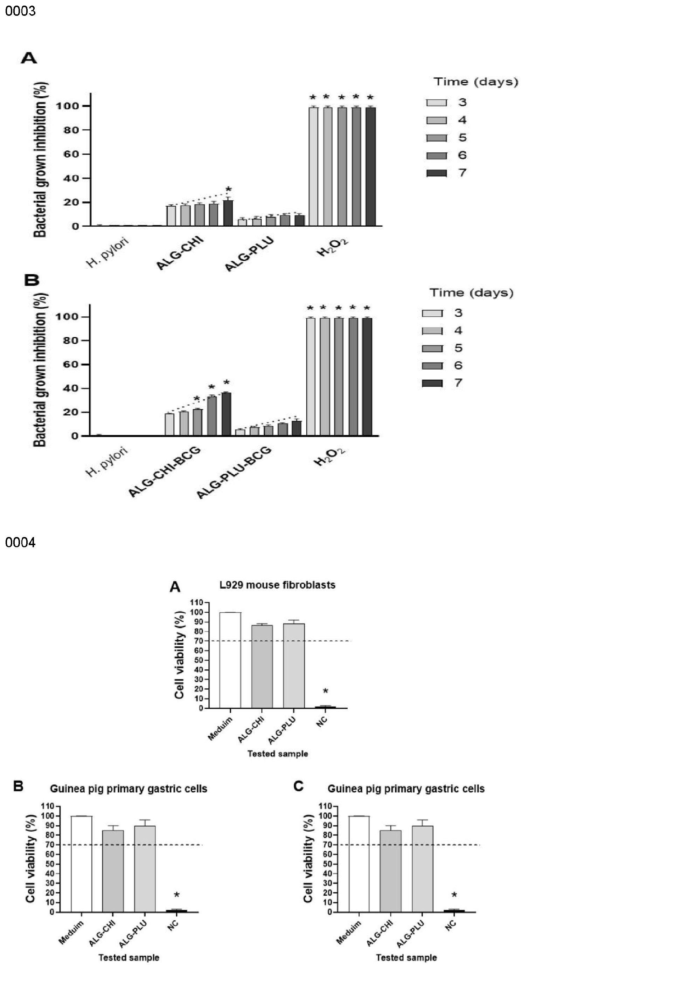

[Fig. 3] shows an *in vitro* assessment of biocidal properties against *Helicobacter pylori;* in Fig. 3A for ALG-CHI (alginate-chitosan) microparticles, ALG-PLU (alginate-Pluronic) microparticles; in Fig. 3B for ALG-CHI-BCG (alginate-chitosan-M. *bovis BCG*) and ALG-PLU-BCG (alginate-Pluronic-M. *bovis* BCG) microparticles
[Fig. 4] shows the *in vitro* biocompatibility of unloaded ALG-MC (alginate): ALG-PLU (alginate-Pluronic), ALG-CHI (alginate-chitosan) in a volume of 100 µL, in the MTT reduction assay;

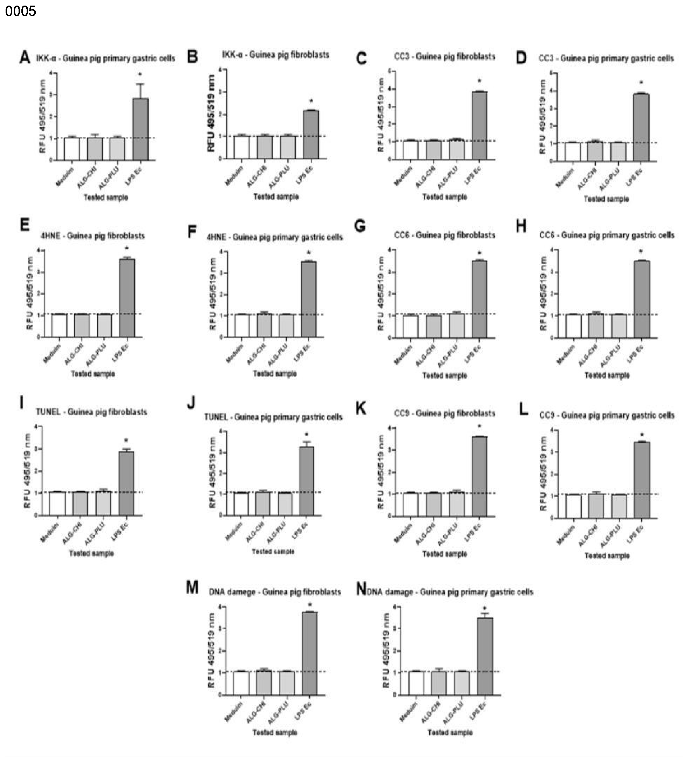

[Fig. 5] shows the assessment of pro-apoptotic activity and DNA damage *in vitro* by unloaded ALG-MC, ALG-PLU and ALG-CHI, in a volume of 100 µL;

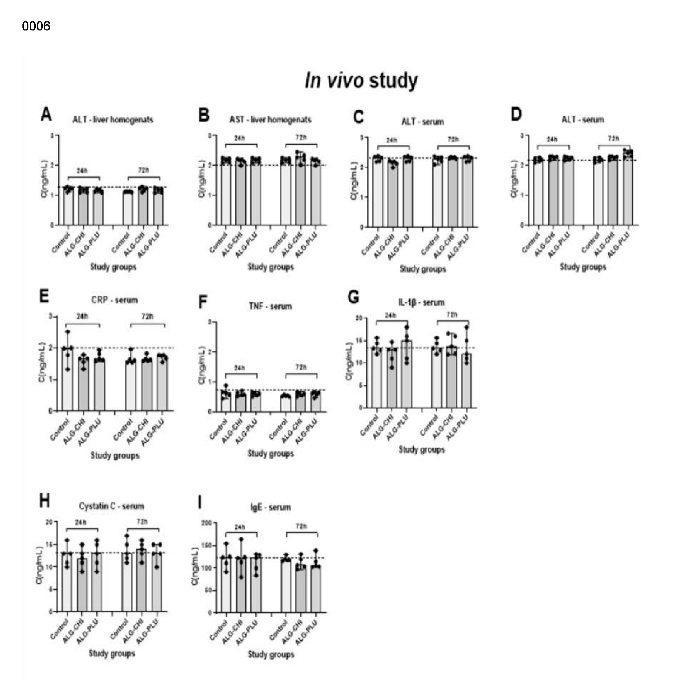

[Fig. 6] shows the *in vivo* biocompatibility assessment of the tested unloaded ALG-MC, ALG-CHI-PLU (alginate-chitosan-Pluronic) and ALG-CHI following oral administration to guinea pigs.

### Example 1

The alginate-based microparticles were produced using a microencapsulation method. They were prepared using commercially available sodium alginate (C₆H₇NaO₆)n of varying molecular weights, a non-ionic copolymer surfactant (C₃H₆O.C₂H₄O)x, and chitosan.

A 2% aqueous solution of sodium alginate was prepared, and this solution was then added dropwise using a 2 mL syringe to a vessel containing a 0.5% calcium chloride solution. The mixture was incubated on a magnetic stirrer for 30 minutes to an hour. The resulting microcapsules were then drained using a laboratory sieve, rinsed with sterile distilled water and transferred to vessels containing, respectively: 1) A 1% solution of chitosan in 1% acetic acid, 2) A 1% solution of Pluronic^{®} F-127 in distilled water. The microcapsules (MC) were incubated in a solution of chitosan or Pluronic^{®} F-127 for 1 hour on a magnetic stirrer. After one hour, the microcapsules were drained using a laboratory sieve and rinsed with sterile distilled water. Only previously sterilised solutions were used during the experiment.

To encapsulate *M. bovis* BCG, 1 mL of a suspension of BCG vaccine bacilli was added to 9 mL of a 2% aqueous alginate solution. The amount of BCG in each sample was 3x10⁹ CFU (1 mL of mycobacterial suspension at this concentration was used). The bacteria were labelled with a fluorescent dye using the BacLight technique (Thermo Fisher Scientific, Waltham, MA, USA). The solution was stirred carefully until the mycobacteria were evenly dispersed throughout the volume. The subsequent steps were carried out in accordance with the procedure described earlier.

Prior to microencapsulation, the alginate was dissolved in water to prepare a homogeneous 2% solution of the substance; for the encapsulation of *M. bovis* BCG, the ingredients were mixed until a suitable dispersion of the bacilli was achieved. The amount of BCG in each sample was 3x10⁹ CFU (1 mL of mycobacterial suspension at this concentration was used). The bacteria were labelled with a fluorescent dye using the BacLight technique (Thermo Fisher Scientific, Waltham, MA, USA).

Microcapsules were obtained consisting of (1) alginate and chitosan, and (2) alginate and Pluronic^{®} F-127. An alginate solution was used to encapsulate *M. bovis* BCG during the microencapsulation process. The resulting microparticles loaded with *M. bovis* BCG were then coated with chitosan or Pluronic-F127. The resulting products were analysed using Fourier transform infrared spectroscopy (FTIR) and a scanning electron microscope (SEM).

### Example 2

Assessment of the viability of *M. bovis* BCG bacilli encapsulated in alginate (ALG) microcapsules.

ALG-Pluronic or ALG-Chitosan microcapsules were prepared as described in Example 1 for solutions, with the molecular weight of the chitosans ranging from 50.000-190.000 g/mol, 190.000-310.000 g/mol, or 310.000-375.000 g/mol, whilst the concentration of the polymer solutions used to prepare the alginate microparticles ranges from 0.25% to 2% by volume. The concentration of the non-ionic copolymer surfactant (C₃H₆O.C₂H₄O)x is 12 600 g/mol.

Next, 3x10⁹ CFU/mL of live *M. bovis* BCG bacilli, previously labelled for 30 minutes with the fluorescent dye BacLight (ThermoFisher, Waltham, MA, USA), were added to each type of solution, and microcapsules (MC) were prepared as described in Example 1. Next, the intensity of the green fluorescence of the labelled bacteria was measured against a previously prepared standard curve for these bacteria in the range of 1x10⁸ CFU/mL, separately for each pH. Fluorescence intensity was measured using a SpectraMax i3 multi-function microplate reader (Molecular Devices, San Jose, CA, USA), with an excitation wavelength of 495 nm and an emission wavelength of 519 nm. In addition, ALG-MC cells containing *M. bovis* BCG bacilli were visualised under a confocal microscope (LSI, Leica) at a wavelength of 495 nm (excitation) and 519 nm (emission), at 4× magnification. The presence of live *M. bovis* BCG bacilli was demonstrated in the MCs prepared.

### Example 3

To assess the *in vitro* activity of empty ALG-MCs (alginate microparticles) and microparticles loaded with *M. bovis* BCG against *Helicobacter pylori,* 100 µL of MCs-with or without mycobacteria-were suspended in 100 µL of *Brucella* medium supplemented with 10% serum. *H. pylori* bacteria were labelled with a fluorescent dye using the BacLight technique (Thermo Fisher Scientific, Waltham, MA, USA), as described previously. A suspension of *H. pylori* (previously labelled) was adjusted to a concentration of 1.5x10⁸ CFU/mL and 100 µL was added to a well in a 96-well plate. Next, empty ALG-MCs or microparticles loaded with *M. bovis* BCG were added to the well. On days 3, 4, 5, 6 and 7, the viability of *H. pylori* was assessed based on the intensity of green fluorescence, which was measured using a SpectraMax i3 multi-function microplate reader (Molecular Devices, San Jose, CA, USA), with an excitation wavelength of 495 nm and an emission wavelength of 519 nm. It has been shown that the addition of chitosan to ALG-MCs inhibits the growth of *H. pylori* by 24% after 7 days, whereas the encapsulation of *M. bovis* BCG in ALG-MCs with chitosan increases the inhibition of *H. pylori* growth to 52% after 7 days.

### Example 4

To determine the release of viable *M. bovis* BCG from MCs, 100 µg of MCs containing mycobacteria were suspended in 100 µL of pre-prepared solutions with different pH levels: alkaline (pH 8.0), neutral (pH 7.2) or acidic (pH 3.0). Before loading *M. bovis* BCG into the MCs, the bacilli were labelled with the fluorescent dye BacLight, as described above, and microencapsulation was used to produce particles containing *M. bovis* BCG , as described in Example 1. Next, each type of MC-BCG, in a solution of the appropriate pH, was dispensed into a 96-well plate in 100 µL volumes. The absorbance was measured for each pH variant at an excitation wavelength of 495 nm and an emission wavelength of 519 nm, at intervals appropriate for each solution's pH, determined on the basis of the dye's stability across different pH ranges, every 10 minutes. A suspension of unencapsulated bacilli of *M. bovis* BCG in PBS at pH 7.2, PBS at pH 3.0, or PBS at pH 8.0 was used as a control. The number of mycobacteria released from ALG-CHI-BCG or ALG-PLu-BCG MCs per 1 mL at the test pH was calculated on the basis of a previously prepared standard curve of labelled bacteria in the range of 1x10⁸ CFU/mL. It has been shown that the addition of a non-ionic copolymer surfactant - (C₃H₆O.C₂H₄O)x - to MCs ensures the highest survival rate of *M. bovis BCG* in PLU-MCs, with a BCG survival rate of 96%; the addition of chitosan ensures a survival rate of *M. bovis* BCG of approximately 95%.

The PBS was adjusted to the final desired pH using 0.1% HCl or 0.1% NaOH. It was demonstrated that live *M. bovis* BCG previously encapsulated in ALG-MCs were released from the microparticles: from ALG-CHI-BCG MCs within 10 to 30 minutes at pH 3.0, pH 7.0, pH 8.0, at a maximum concentration of 1,76x10⁸ CFU/mL within 20 minutes, whilst from ALG-PLU-BCG MCs between 10 and 30 minutes, at pH 3.0, pH 7.0, pH 8.0, at a maximum concentration of 1.83x10⁸ CFU/mg, within 20 minutes.

### Example 5

Evaluation of alginate microcapsules for their cytotoxic, genotoxic and pro-apoptotic properties.

*In vitro* biocompatibility: Empty MC: ALG-PLU and ALG-CHI, in a volume of 100 µL, were tested using (3-(4,5-dimethylthiazol-2-yl)-2,5-diphenyl tetrazolium bromide) (MTT) in accordance with ISO 10993-5 Biological evaluation of medical devices - Part 5: *In vitro* cytotoxicity tests ; International Organisation for Standardisation, 2009.

For this purpose, the recommended L929 mouse fibroblasts, primary guinea pig gastric epithelial cells or fibroblasts were used. No cytotoxic activity was observed for the microparticle variants tested in the MTT reduction assay without *M. bovis* BCG.

DNA damage *in vitro:* Empty MC: 100 µL of ALG-PLU or ALG-CHI, or *E. coli LPS* (as a positive control), was added to primary cultures of gastric epithelial cells or guinea pig fibroblasts and incubated for 24 hours. DNA damage was assessed using the HCS DNA Damage Kit (Thermo Fisher Scientific, Waltham, MA, USA), in accordance with the manufacturer's instructions. DNA damage has been identified by an antibody against phosphorylated H2AX (Ser139), which enables the detection of double-strand breaks (DSBs). No genotoxic effects were observed in the tested MPs variants without the addition of *M. bovis* BCG.

Induction of apoptosis *in vitro:* Empty MC: 100 µL of ALG-PLU. ALG-CHI, or *E*. *coli LPS* (as a positive control), was added to primary cultures of gastric epithelial cells or guinea pig fibroblasts and incubated for 24 hours. The samples were then stained immunohistochemically for the presence of early pro-apoptotic caspase 3 (CC3) or caspase 9 (CC9) associated with the intermediate stage of apoptosis, as well as the late apoptosis marker carbamoyl which is carbamoyl phosphate synthetase-2 aspartate transcarbamylase-dihydroorotase (CAD protein). The staining procedure was carried out using fluorescently labelled primary rabbit antibodies (Santa Cruz Biotechnology, Dallas, TX, USA), followed by a secondary antibody against rabbit immunoglobulins, Alexa Fluor 488-IgG (Invitrogen, Waltham, MA, USA). The concentrations of CC3, CC9 and CAD were determined by measuring the fluorescence intensity at an excitation wavelength of 495 nm and an emission wavelength of 519 nm, using a SpectraMax i3 multi-parameter reader (Molecular Devices, San Jose, CA, USA). No pro-apoptotic activity was observed in the tested MPs variants in the absence of *M. bovis* BCG.

### Example 6

Assessment of the *in vivo* biodistribution of chitosan microparticles.

The study was conducted on animals - domestic cavy/guinea pigs - following approval from the Ethics Committee for Animal Experimentation at the Medical University of 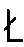 ódź, appointed by the Minister of Science and Higher Education (Approval No. 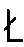 B 16/234/2022).

The guinea pigs were inoculated *per os* with 1 mL of empty ALG-MCs, ALG-PLU and ALG-CHI, each in a volume of 100 µL in 0.85% NaCl. The control animals were given 1 mL of 0.85% NaCl. The MC has been prepared in accordance with standard PN EN ISO 10993-12:2012E. 24, 48 or 72 hours after administration of MCs, the animals were euthanised by an overdose of sodium barbiturate (Morbital, Biowet, Putawy, Poland), after which blood and organs (spleen, liver, kidneys) were collected and examined to rule out systemic and tissue disorders.

In all animals, the concentrations of alanine aminotransferase (ALT) and aspartate aminotransferase (AST) in liver homogenates and serum samples were determined using a commercial ELISA test (MyBiosource, San Diego, USA) with a sensitivity of 0.06 ng/mL and < 0.118 ng/mL, respectively, in accordance with the attached protocol.

In addition, serum levels of pro-inflammatory cytokines-tumour necrosis factor alpha (TNF-α) and interleukin (IL)-1B-were measured using an ELISA (Thermo Fisher Scientific, Waltham, MA, USA) with a sensitivity of 1.7 pg/mL (TNF-α) and 1 pg/mL (IL-1β), respectively, in accordance with the manufacturer's instructions. In addition, serum IgE levels, cystatin C and C-reactive protein (CRP) were measured with a sensitivity of: 0.14 ng/mL (IgE), 10 pg/mL (cystatin C), 0.15 ng/mL (CRP), 1.7 pg/mL (TNF-α) and 1 pg/mL (IL-1β), respectively, in accordance with the manufacturer's instructions. The liver, kidneys and spleen were stained using standard haematoxylin and eosin staining to rule out any pathological changes in the organs.

In animals receiving the test variants of MCs without the addition *of M. bovis* BCG, no deviation was observed in the values of the test parameters compared with those in the control animals.

### BIBLIOGRAPHY

**[1]** Hegde, V., Uthappa, U. T., Altalhi, T., Jung, H. Y., Han, S. S., & Kurkuri, M. D. (2022) Alginate based polymeric systems for drug delivery, antibacterial/microbial, and wound dressing applications. Materials Today Communications, 33, 104813.
**[2]** Fernando, I. P. S., Lee, W., Han, E. J., & Ahn, G. (2020). Alginate based nanomaterials: Fabrication techniques, properties, and applications. Chemical Engineering Journal, 391, 123823.
**[3]** Goh, C. H., Heng, P. W. S., & Chan, L. W. (2012). Alginates as a useful natural polymer for microencapsulation and therapeutic applications. Carbohydrate Polymers 88, 1-12.
**[4]** Aldwell F. E., Tucker I. G., de Lisle G. W., Buddle B.M. (2003) Oral Delivery of Mycobacterium bovis BCG in a Lipid Formulation Induces Resistance to Pulmonary Tuberculosis in Mice, Infection and Immunity, 71, 101-108; Cross M.L., Lambeth M.R., Coughlan Y., Aldwell F.E. (2007) Oral vaccination of mice with lipid-encapsulated Mycobacterium bovis BCG: Effect of reducing or eliminating BCG load on cell-mediated immunity, Journal of Microencapsulation, 25, 1297-1303.
**[5]** Dobakhti F., Rahimi F., Dehpour A. R., Taghikhani M., Ajdary S., Rafiei S., Rafiee-Tehrani M. (2006) Stabilizing effects of calcium alginate microspheres on mycobacterium bovis BCG intended for oral vaccination, Journal of Microencapsulation, 23(8), 844-854.
**[6]** Rosevear, H. M., Lightfoot, A. J., O'Donnell, M. A., & Griffith, T. S. (2009) The role of neutrophils and TNF-related apoptosis-inducing ligand (TRAIL) in bacillus Calmette-Guérin (BCG) immunotherapy for urothelial carcinoma of the bladder. Cancer and Metastasis Reviews, 28, 345-353.
**[7]** Covián C., Fernández-Fierro A., Retamal-Diaz A., Díaz F. E., Vasquez A. E., Lay M. K., Riedel C. A., González P. A., Bueno S. M., Kalergis A. M. (2019) BCG-Induced Cross-Protection and Development of Trained Immunity: Implication for Vaccine Design.Frontiers in Immunology, 10, 2806.

## Claims

1. A method of manufacturing microparticles containing *Mycobacterium bovis* BCG vaccine bacilli encapsulated in an alginate carrier, intended for combating *Helicobacter pylori* infection, **wherein** an aqueous solution of a non-ionic copolymeric surfactant (C₃H₆O.C₂H₄O)x and/or a solution of chitosan in 1% acetic acid is introduced into an aqueous alginate solution, followed by a microencapsulation procedure involving the dropwise addition of the aforementioned solutions, using a 2 mL syringe, to a vessel containing a 0.5% calcium chloride solution, at room temperature on a magnetic stirrer for 1 hour, wherein the molecular weight of the chitosans is in the range of 50.000-190.000 g/mol or 190.000-310.000 g/mol or 310.000-375.000 g/mol, wherein the concentration of the polymer solutions for the preparation of microcapsules is 2% v/v for alginate, 1% v/v for chitosan and 12 600 g/mol for the non-ionic copolymer surfactant (C₃H₆O.C₂H₄O)x, respectively.

2. Method according to claim 1, **wherein** live M. *bovis* BCG are suspended in 1 mL of saline solution, with a BCG concentration of 3x10⁹ CFU/mL.
